# EUROPEAN PATENT SPECIFICATION

(11) **EP 2 301 445 B1**
(45) Date of publication and mention of the grant of the patent: **22.08.2012**
(21) Application number: 10184681.4
(22) Date of filing: 20.10.2001
(51) Int. Cl.: A61B 17/00, A61B 17/02, A61B 17/34

(54) **Wound retraction apparatus**
Wundretraktionsvorrichtung
Appareil de retraction de plaies

(43) Date of publication of application: 30.03.2011
(62) Divisional of application: 01985170.8
(73) Proprietor: APPLIED MEDICAL RESOURCES CORPORATION, Rancho Santa Margarita, CA 92688 (US)
(72) Inventor: Ewers, Richard C, Fullerton, CA 92833 (US); Brustad, John R, Dana Point, CA 92629 (US); Pingleton, Edward D, San Juan Capistrano, CA 92675 (US); Hilal, Nabil, Laguna Niguel, CA 92677 (US); Adlparvar, Payam, Lake Forest, CA 92630 (US); Taylor, Scott, Mission Viejo, CA 92692 (US); Dulak, Gary R, Newport Beach, CA 92663 (US); Dunn, Michael J, Santa Ana, CA 92706 (US); Morales, Norman, San Jose, CA 95126 (US); Hart, Charles C, Summerville, SC 29483-8949 (US); Bowes, Robert R. II, Trabuco Canyon, CA 92679 (US)
(74) Representative: Fitchett, Stuart Paul

(56) References cited:
- EP-A- 1 125 552
- WO-A-00/32116
- WO-A-01/45568
- WO-A1-98/48724
- US-A- 5 524 644

## Description

### Background of the Invention

### Field of Invention

This invention relates generally to wound retraction and more specifically to wound retraction in a laparoscopic surgical procedure.

### Discussion of the Related Art

During laparoscopic surgery, it is desirable to inflate the abdominal cavity in order to increase the volume of the working space. This is accomplished with an insufflation gas which must be maintained at a pressure sufficient to inflate the abdomen. Maintaining the pressure of the insufflation gas is difficult when it is also desirable to insert instrumentation through the abdominal wall. If the surgeon is interested in inserting his or her hand in a hand-assisted laparoscopic procedure, the maintenance of insufflation pressure is even more difficult. Currently, several devices exist that accomplish this surgical need although they suffer from drawbacks such as difficult placement and cumbersome use. Thus, it is desirable that the wound be retracted, protected, and fixed while maintaining an insufflation seal. European patent application number EP 1,125,552 discloses a surgical retractor for retracting the margins of a wound, and PCT application number WO 00/32116 discloses a surgical device for both retracting and protecting a wound.

### Summary of the Invention

Wound retraction in accordance with the present invention allows the surgeon to easily locate a retractor and to provide a solid base for an instrument or hand seal. This retractor removes the tissue pressure from the wrist during hand-assisted laparoscopic surgery. It can also protect the tissue at the wound site, for example, from abrasion, bacteria or other contaminants organs, such as donor kidneys to be removed with minimal risk or damage. The retractor also opens the wound providing greater access to the operative site for instruments, such as the hand of the surgeon.

According to the present invention, there is provided a surgical wound retractor as recited in Claim 1. The dependent claims disclose preferable but non-essential features.

These and other features and advantageous of the invention will become more apparent with a description of preferred embodiments and reference to the associated drawings.

### Description of the Drawings

FIGS. 1 and 2 are perspective views illustrating the principle of operation of a wound retractor;
FIG. 3 is an axial perspective view of a surgical wound retractor including a third ring in accordance with the present invention;
FIG. 4A is an axial schematic view showing the FIG. 3 embodiment in a natural state;
FIG. 4B is an axial schematic view showing the FIG. 3 embodiment in a retraction state;
FIG. 5 - FIG. 8 are axial perspective views showing steps in a preferred method for operating the FIG. 3 embodiment;

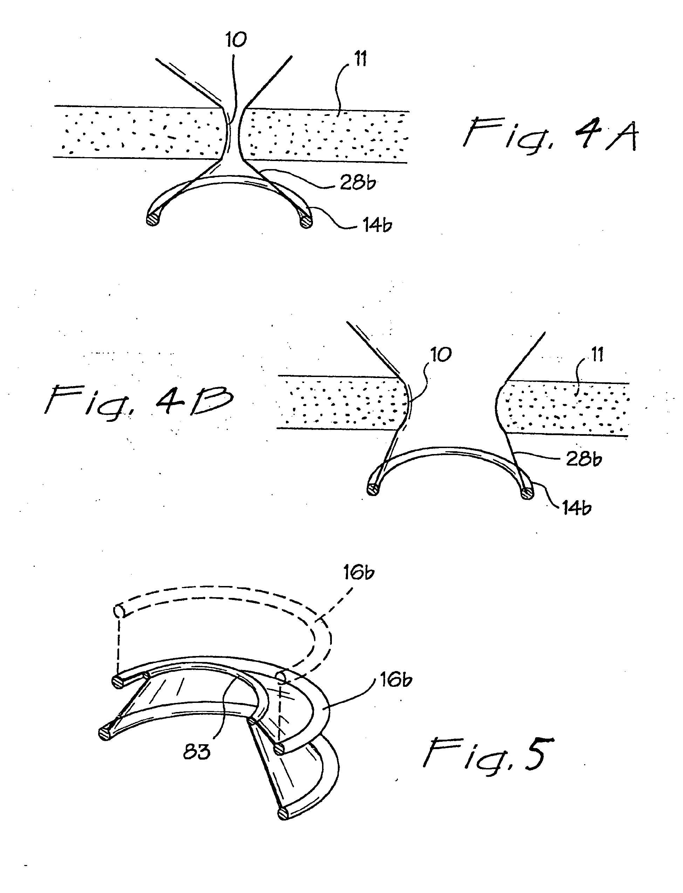

FIG. 5 is an axial perspective view showing the second ring being rolled over the third ring to create a circumferential retainer;

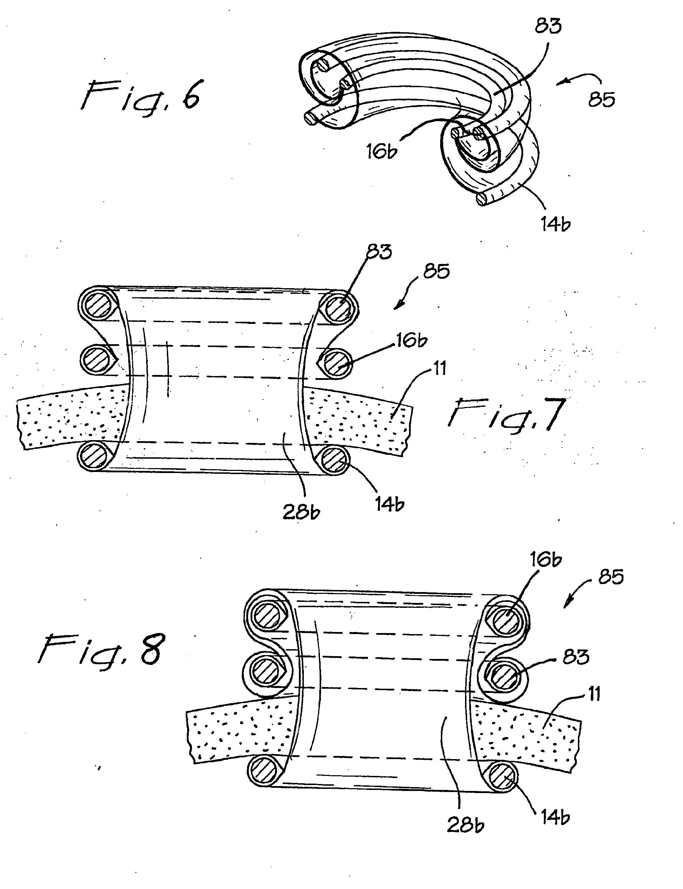

FIG. 6 is an axial perspective view showing the third ring being rolled over the second ring to move the circumferential retainer to a desired position;
FIG. 7 is an axial view showing the step of Figure 5 with the retractor operatively disposed;
FIG. 8 is an axial cross section view of the step illustrated in FIG.6 showing the retractor operatively disposed;

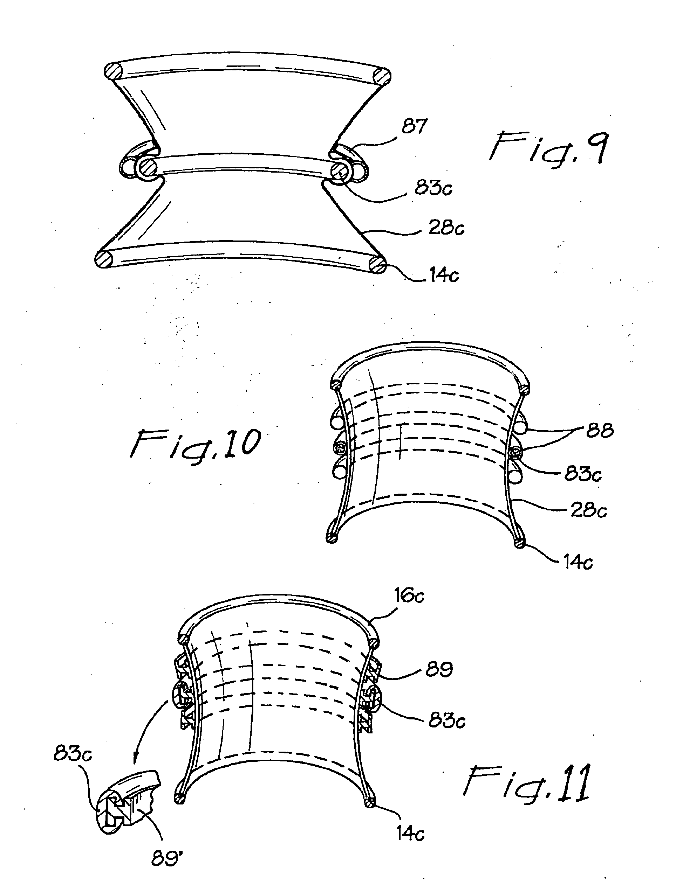

FIG. 9 is an axial perspective view of a further embodiment including a clip for retaining the third ring at a desired position between the second ring and the first ring;
FIG. 10 is an axial perspective view showing multiple loops each disposed at a particular distance from the first ring and adapted to alternatively receive the third ring at a desired distance from the first ring;
FIG. 11 is an axial perspective view of an embodiment including tracks and a third ring with a mating groove;

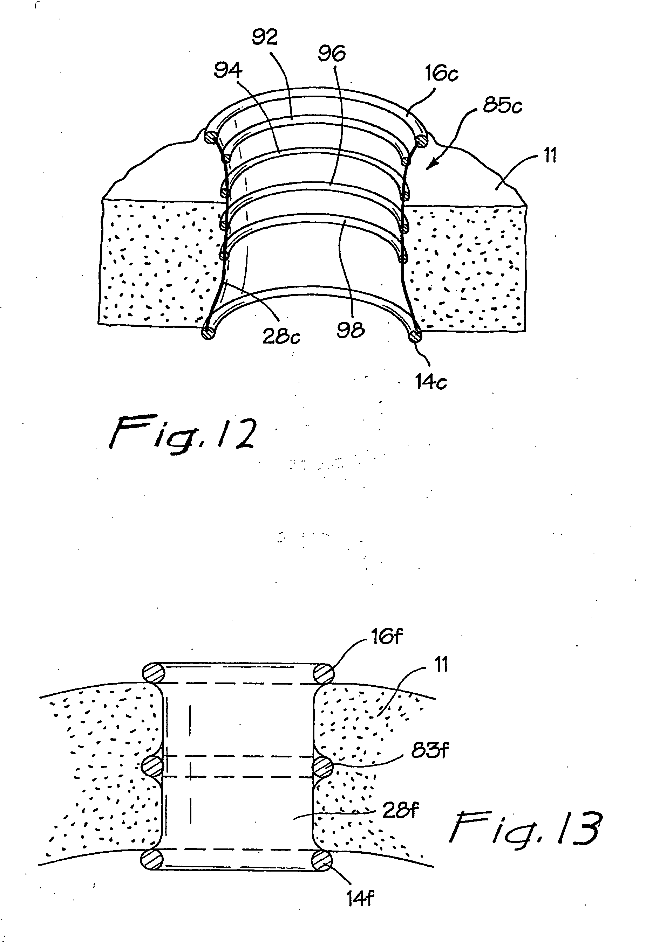

FIG. 12 is an axial perspective view of a further embodiment including a series of third rings for forming the retention roll at different positions between the first and second rings;
FIG. 13 is an axial cross section view illustrating an embodiment with two end rings and at least one intermediate ring.

### Description of Preferred Embodiments and Best Mode of the Invention

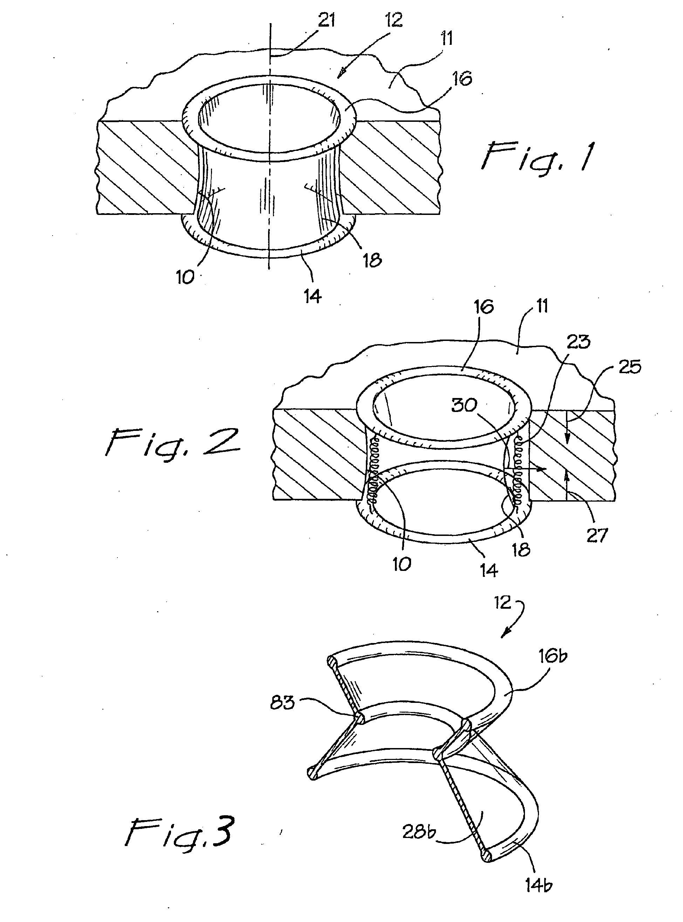

The basic concept of retracting and protecting a wound site is illustrated in the prospective view of Figure 1 wherein a wound 10 is formed in an abdominal wall 11. In this embodiment, a retractor 12 uses two rings 14 and 16 which are fixed to an elastic sheath 18. The sheath 18 has a generally cylindrical configuration and is disposed along an axis 21. The rings 14 and 16 are disposed in respective planes which extend radially of the axis 21.

The sheath 18 has elastomeric properties, but in its natural, unstretched state the two rings 14 and 16 are separate by a natural distance. The lower ring 14 is placed interiorly of the abdominal wall 11 and the upper ring 14 is stretched beyond the natural distance away from the lower ring. Once the elastic sheath 18 has been stretched to a distance greater than the abdominal wall thickness, the upper ring 16 is placed on the surface of the skin.

Since the diameters of the rings 14, 16 are greater than that desired for the wound site 10, they will have sufficient footing to maintain this tension between the two rings 14, 16. This tension is created by the elastic material that has been stretched and retained at a distance greater than the natural distance. It will be appreciated that in many embodiments, the sheath 18 can be formed of a non-elastic sheathing material. In a similar manner, the rings 14 and 16 may be provided with a rigid configuration or alternatively may be formed of an elastromeric material.

Figure 2 shows a simple schematic of the ring dynamics illustrated in Figure 1, with the retractor 12 operatively disposed across the abdominal wall 11. The elastic sheet sheath 18 acts as a circumferential spring 23 around the wound site 10 that evenly distributes the tension between the two rings 14 and 16, as represented by arrows 25 & 27. In addition, the elastic sheeting provides a radial retraction force 30 around the wound to enlarge the wound site 10 in order to facilitate the passage of instruments, such as the hand of the surgeon.

The amount of tension force between the two rings 14 and 16 can be controlled by the elastomeric proportion of the elastic sheath 18. In order to accommodate a larger range of abdominal wall thicknesses, a material with a higher elasticity can be chosen to allow for greater stretch.

Embodiments of retractors 12 in accordance with the present invention are illustrated in Figures 3-13. Referring to Figure 3, in this embodiment, the retractor 12 includes the thin film sheath 28b which lines the wound to prevent or limit the risk of portsite metastasis. The inner and outer rings, 14b and 16b, respectively, are attached to opposing ends of the sheath 28b in the manner previously described. However, in this embodiment, a third ring 83 is fixed to the sheath 28b between the rings 14b and 16b.

In order to achieve the desired retraction, the inner ring 14b is positioned interiorly of the abdominal wall 11 as illustrated in Figure 4A. Then the sheath 28b is pulled outwardly through the wound 10, tensioning the sheath 28b and causing the abdominal wall 11 to retract or open as illustrated in Figure 4B.

At this point, it is desirable to maintain the retraction by preventing the sheath 28b from pulling back into the wound 10. This maintenance of tension on the sheath 28b is addressed in a unique manner with the embodiment of Figure 3 wherein the rings 16b and 83 are provided with elastromeric characteristics.

In the manner illustrated in Figure 5, the ring 16b can be moved distally over the ring 83. This creates a circumferential retainer or ring bundle 85 which is illustrated in the perspective view of Figure 6. In this process, the ring 16b is stretched circumferentially outwardly of the ring 83 to form the bundle 85. This formation is further illustrated in the cross section schematic view of Figure 7 where the bundle 85 of elastomeric rings presses against the outer surface of the abdominal wall 11 to maintain tension on the sleeve 28b. With reference to Figure 8, it will be appreciated that the ring bundle 85 can be further rotated to move the third ring 83 outwardly and distally of the ring 16b as required to produce and maintain the desired tension on the sheath 28b.

With the embodiment of Figure 3, retraction is achieved by positioning the ring 83 above the ring 16b, and pulling the sheath 28b upwardly from the wound 10 (FIG. 5). The sheath is prevented from pulling back into the incision by stretching the ring 83 out, around, and under the ring 16b.

The degree of retraction or how much the sheath is pulled upwardly, is depended on the height of the ring 83 about the abdominal wall 11. This height can be adjusted in the embodiment of Figure 9 wherein elements of structure similar to those previously described are designated with the same reference numeral followed by the lower case letter "c." In this embodiment, the ring 83c can be disconnected from and moved relative to the sheath 28c. At a desired location above the inner ring 14c, the ring 83c can be fixed to the sheath 28c, for example by a clip 87.

Clamps or clips may be used to lock the movable ring at the appropriate height as shown in Figure 9. Also, a series of open loops 88 (FIG. 10) or tracks 89 (FIG. 11) can be fixed to the sheath 28c at different distances from the inner ring 14c to hold the ring 83c at different heights.

In still a further embodiment, illustrated in Figure 12, several intermediate rings, 92, 94, 96 and 98 can be provided between the inner ring 14c and the outer ring 16c. With this embodiment, the rings, such as the ring 16c, 92 and 94, disposed outwardly of the abdominal wall 11 can be rolled into the ring bundle 85c to maintain the desired tension on the sheath 28c. In this case, the rings 96 and 98, which are not included in the ring bundle 85c, contact the abdominal wall 11 to provide increased friction and perhaps increased retraction at the wound site.

A further advantage of these embodiments is that they enable the surgeon to retract and protectively cover the wound 10 while permitting adjustment of the retractor 12c to variations in thickness of the abdominal wall 11. These devices provide an airtight seal around the wound 10, thus allowing an airtight cap or access port to be attached to enable laparoscopic surgery.

In any of the embodiments previously disclosed, a sealing cap can be disposed over the outer ring 16b to facilitate laparoscopic surgery.

A further embodiment of the wound retractor is, illustrated in Figure 13, where elements of structure similar to those previously disclosed are designated with the same reference numeral followed by the lower case letter "f." This embodiment includes three separate rings 16f, 14f, and 83f. The device has a tubular sheath 28f which lines the incision to prevent or limit the risk of portsite metastasis. The device includes the external ring 16f attached to the proximal end of the sheath 28f, the intermediate ring 83f attached to the center of the sheath, and a peritoneal ring attached to the distal end of the sheath 28f. The peritoneal ring is designed to be placed inside the incision while the eternal ring 16f remains external to the incision. The intermediate ring 83 f may remain external to the incision or may lie within the incision itself depending upon how the three rings are oriented.

This wound retractor has four different fixed lengths to accommodate abdominal walls of four different thicknesses. The first length, which is the longest, is achieved by simply placing the peritoneal ring 14f into the incision. The tension of the sheath between the external ring 16f and the peritoneal ring 14f maintains the incision in an open configuration. If the thickness of the abdominal wall 11 is less than the distance between the eternal ring 16f and the peritoneal ring 14f, then a shorter length can be selected.

The second length, which is less than the first length, is achieved by simply placing the peritoneal ring 14f into the incision, and then pulling the sheath 28f upwards until the intermediate ring 83f is external to the incision. The tension of the sheath 28f between the intermediate ring 83f and the peritoneal ring 14f maintains the incision in an open configuration. If the thickness of the abdominal wall 11 is less than the distance between the intermediate ring 83f and the peritoneal ring 14f then a shorter length can be selected.

The third length, which is less than the second length, is achieved by inserting the peritoneal ring 14f into the incision and then pulling the intermediate ring 83f over the external ring 16f. The tension of the sheath between the peritoneal ring and the combination of the external ring and the intermediate ring maintains the incision in an open configuration. If the thickness of the abdominal wall is less than the third length, then the fourth length will have to be selected.

The fourth length, which is less than the third length, is achieved by first pulling the intermediate ring 83f through the external ring 16f, and then pulling the intermediate ring 83f over the external ring 16f. The tension of the sheath 28f between the peritoneal ring 14f and the combination of the external ring 16f and the intermediate ring 83f, maintains the incision in an open configuration. This 3-ring retractor can therefore be positioned to effect four different lengths to accommodate variations in the thickness of the abdominal wall from patient to patient.

A most significant advantage associated with this embodiment is that the device enables a surgeon to retract and protectively line an abdominal wall incision while being able to easily adjust the device to accommodate variations from patient to patient in the thickness of the abdominal wall. The device is also very low in cost since it includes only four components, 3 rings 14f, 16f, and 83f, and a tubular sheath 28f.

It will be understood that many other modifications can be made to the various disclosed embodiments without departing from the scope of the invention as claimed. For example, various sizes of the surgical device are contemplated as well as various types of constructions and materials. It will also be apparent that many modifications can be made to the configuration of parts as well as their interaction. For these reasons, the above description should not be construed as limiting the invention, but should be interpreted as merely exemplary of preferred embodiments. Those skilled in the art will envision other modifications within the scope of the present invention as defined by the following claims.

## Claims

1. A surgical wound retractor, adapted to dilate a wound, the retractor comprising:
a first ring (14b) having a diameter greater than the desired diameter of the wound and being adapted for disposition interiorly of the wound;
a second ring (16b) adapted for disposition exteriorly of the wound; and
a retraction sleeve (28b) having a generally cylindrical configuration with a first end coupled to the first ring and a second end coupled to the second ring;
**characterized in** the retractor further comprises
a third ring (83) coupled to the sleeve in a fixed relationship between the first end and the second end, the third ring being adapted for disposition exteriorly of the wound; and
the second ring and the third ring having elastomeric properties facilitating the second ring (16b) to be moved distally over the third ring (83) to form a ring bundle (85) and the ring bundle to be further rotated to move the third ring (83) outwardly and distally of the second ring (16b) to provide the sleeve with a radial retraction force sufficient to dilate the wound to the desired diameter.

2. The retractor of claim 1, wherein the third ring (83c) can be located at different positions on the sleeve (28c) and is arranged to be fixed to the sleeve (28c) by a clip (87) or clamp prior to the sleeve (28c) being tensioned.

3. The retractor of claim 1, comprising a series of open loops (88) or tracks (89) fixed to the sleeve (28c) at different distances from the first ring (14c) to hold the third ring (83c) at different positions on the sleeve (28c).

4. The retractor of claim 1, comprising a plurality of third rings (92, 94, 96, 98) fixed to the sleeve (28c) between the first ring (14c) and the second ring (16c).

5. The retractor any of claims 1 to 4, wherein the second (16b) and third (83) rings are spaced apart and have a portion of the sleeve extending between them.

6. The retractor of any preceding claim, further comprising a sealing cap (105) disposed over at least one of the second ring (16b) and the third ring (83).

7. The retractor of claim 6, wherein the sealing cap is attachable to at least one of the second ring (16b) and the third ring (83).

8. The retractor of any of any preceding claim, wherein the retraction sleeve (28b) is a thin film sheath.

## Patentansprüche

1. Ein chirurgischer Wundretraktor, der zur Dilatation einer Wunde angepasst ist, wobei der Retraktor umfasst:
einen ersten Ring (14b) mit einem Durchmesser, der größer ist als der gewünschte Durchmesser der Wunde und der zur Anordnung innerhalb der Wunde angepasst ist;
einen zweiten Ring (16b), der zur Anordnung außerhalb der Wunde angepasst ist; und
eine Rückhalteschleuse (28b), die eine allgemein zylinderförmige Konfiguration aufweist, wobei ein erstes Ende an den ersten Ring gekoppelt ist und ein zweites Ende an den zweiten Ring gekoppelt ist;
**gekennzeichnet dadurch, dass** der Retraktor weiterhin umfasst
einen dritten Ring (83), der in einer festen Beziehung zwischen dem ersten Ende und dem zweiten Ende an die Schleuse gekoppelt ist, wobei der dritte Ring zur Anordnung außerhalb der Wunde angepasst ist; und
der zweite Ring und der dritte Ring elastomere Eigenschaften aufweisen, die es ermöglichen, den zweiten Ring (16b) distal über den dritten Ring (83) zu bewegen, um ein Ringbündel (85) zu bilden, und das Ringbündel weiter zu drehen, um den dritten Ring (83) nach außen und distal zu dem zweiten Ring (16b) zu bewegen, um der Schleuse eine radiale Rückhaltekraft bereitzustellen, die ausreichend ist, um die Wunde auf den gewünschten Durchmesser zu dilatieren.

2. Der Retraktor nach Anspruch 1, wobei der dritte Ring (83c) sich an verschiedenen Positionen auf der Schleuse (28c) befinden kann und so angeordnet ist, dass er mit einem Clip (87) oder einer Klammer an der Schleuse (28c) befestigt werden kann, bevor die Schleuse (28c) angespannt wird.

3. Der Retraktor nach Anspruch 1, der eine Reihe von offenen Schleifen (88) oder Spuren (89) umfasst, die in verschiedenen Abständen von dem ersten Ring (14c) an der Schleuse (28c) befestigt sind, um den dritten Ring (83c) in verschiedenen Positionen auf der Schleuse (28c) zu halten.

4. Der Retraktor nach Anspruch 1, der eine Vielzahl von dritten Ringen (92, 94, 96, 98) umfasst, die zwischen dem ersten Ring (14c) und dem zweiten Ring (16c) an der Schleuse (28c) befestigt sind.

5. Der Retraktor nach einem beliebigen der Ansprüche 1 bis 4, wobei der zweite (16b) und der dritte (83) Ring im Abstand voneinander angebracht sind und ein Teil der Schleuse sich zwischen ihnen erstreckt.

6. Der Retraktor nach einem beliebigen vorherigen Anspruch, der weiterhin eine Dichtungskappe (105) umfasst, die über mindestens einem von dem zweiten Ring (16b) und dem dritten Ring (83) angeordnet ist.

7. Der Retraktor nach Anspruch 6, wobei die Dichtungskappe an mindestens einem von dem zweiten Ring (16b) und dem dritten Ring (83) befestigt werden kann.

8. Der Retraktor nach einem beliebigen vorherigen Anspruch, wobei die Rückhalteschleuse (28b) eine dünne Filmhülle ist.

## Revendications

1. Écarteur chirurgical, adapté de façon à dilater une plaie, ce rétracteur comprenant :
une première bague (14b) ayant un diamètre plus grand que le diamètre désiré de la plaie et adapté de façon à être disposé à l'intérieur de la plaie ;
une deuxième bague (16b) adaptée de façon à être disposée à l'extérieur de la plaie ; et
un manchon d'écartement (28b) ayant une configuration généralement cylindrique avec une première extrémité attachée à la première bague et une deuxième extrémité attachée à la deuxième bague ;
**caractérisé en ce que** cet écarteur comprend en outre :
une troisième bague (83) attachée au manchon dans un rapport fixe entre la première et la deuxième extrémité, cette troisième bague étant adaptée de façon à être disposée à l'extérieur de la plaie ; et
la deuxième bague et la troisième bague ayant des propriétés élastomères facilitant le déplacement de la deuxième bague (16b) dans une direction distale au-dessus de la troisième bague (83) afin de former un groupe de bagues (85), et ce groupe de bagues devant être encore tourné de façon à déplacer la troisième bague (83) vers l'extérieur et distalement par rapport à la deuxième bague (16b) afin de fournir au manchon une force d'écartement radial suffisante pour dilater la plaie au diamètre désiré.

2. Écarteur selon la revendication 1, dans lequel la troisième bague (83c) peut être placée dans différentes positions sur le manchon (28c) et est agencée de façon à être fixée au manchon (28c) par une pince (87) ou une bride de serrage avant la mise sous tension du manchon (28c).

3. Écarteur selon la revendication 1, comprenant une série de boucles (88) ou de pistes (89) ouvertes fixées au manchon (28c) à différentes distances de la première bague (14c) afin de maintenir la troisième bague (83c) dans différentes positions sur le manchon (28c).

4. Écarteur selon la revendication 1, comprenant une pluralité de troisièmes bagues (92, 94, 96, 98) fixées au manchon (28c) entre la première bague (14c) et la deuxième bague (16c).

5. Écarteur selon l'une quelconque des revendications 1 à 4, dans lequel la deuxième (16b) et la troisième (83) bague sont écartées et ont une partie du manchon s'étendant entre elles.

6. Écarteur selon l'une quelconque des revendications précédentes, comprenant en outre une calotte d'étanchéité (105) disposée au-dessus d'au moins soit la deuxième bague (16b), soit la troisième bague (83).

7. Écarteur selon la revendication 6, dans lequel la calotte d'étanchéité peut être attachée à au moins soit la deuxième bague (16b), soit la troisième bague (83).

8. Écarteur selon l'une quelconque des revendications précédentes, dans lequel le manchon d'écartement (28b) est une gaine en film mince.
